(19) Europäisches Patentamt
European Patent Office
Office européen des brevets

(11) EP 4 186 496 B1

(12) EUROPEAN PATENT SPECIFICATION

(45) Date of publication and mention of the grant of the patent:
**08.10.2025 Bulletin 2025/41**

(21) Application number: **21847141.5**

(22) Date of filing: **19.07.2021**

(51) International Patent Classification (IPC):
***A61K 31/05*** *(2006.01)* ***A61P 31/14*** *(2006.01)*
***A61K 31/00*** *(2006.01)* ***A61P 11/00*** *(2006.01)*
***A61K 45/06*** *(2006.01)* ***A61K 31/13*** *(2006.01)*
***A61K 31/215*** *(2006.01)* ***A61K 31/46*** *(2006.01)*
***A61K 31/4725*** *(2006.01)* ***A61K 31/513*** *(2006.01)*
***A61K 31/522*** *(2006.01)* ***A61K 31/536*** *(2006.01)*
***A61K 31/551*** *(2006.01)* ***A61K 31/662*** *(2006.01)*
***A61K 31/675*** *(2006.01)* ***A61K 31/683*** *(2006.01)*
***A61K 31/7012*** *(2006.01)* ***A61K 31/7056*** *(2006.01)*
***A61K 31/7072*** *(2006.01)* ***A61K 38/21*** *(2006.01)*

(52) Cooperative Patent Classification (CPC):
(C-Sets available)
**A61K 31/658; A61K 31/13; A61K 31/215; A61K 31/46; A61K 31/4725; A61K 31/513; A61K 31/522; A61K 31/536; A61K 31/551; A61K 31/662; A61K 31/675; A61K 31/683; A61K 31/7012; A61K 31/7056; A61K 31/7072;**
(Cont.)

(86) International application number:
**PCT/CN2021/107001**

(87) International publication number:
**WO 2022/017304 (27.01.2022 Gazette 2022/04)**

(54) **APPLICATION OF CANNABIDIOL IN TREATMENT OF CORONAVIRUS INFECTIONS**

VERWENDUNG VON CANNABIDIOL BEI DER BEHANDLUNG VON CORONAVIRUS-INFEKTIONEN

APPLICATION DU CANNABIDIOL DANS LE TRAITEMENT D’INFECTIONS À CORONAVIRUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.07.2020 CN 202010722697**

(43) Date of publication of application:
**31.05.2023 Bulletin 2023/22**

(73) Proprietor: **Academy of Military Medical Sciences**
**Beijing 100850 (CN)**

(72) Inventors:
- **ZHONG, Wu**
  **Beijing 100850 (CN)**
- **QIN, Yong**
  **Beijing 100850 (CN)**
- **WANG, Manli**
  **Beijing 100850 (CN)**
- **CAO, Ruiyuan**
  **Beijing 100850 (CN)**
- **FAN, Shiyong**
  **Beijing 100850 (CN)**
- **HU, Zhihong**
  **Beijing 100850 (CN)**
- **LI, Song**
  **Beijing 100850 (CN)**

(74) Representative: **Weickmann & Weickmann PartmbB**
**Postfach 860 820**
**81635 München (DE)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(56) References cited:
WO-A1-2021/228365 WO-A2-2021/199078
WO-A9-2012/012498 CN-A- 102 924 444
CN-A- 111 686 095

- HESAM KHODADADI ET AL: "Cannabidiol Modulates Cytokine Storm in Acute Respiratory Distress Syndrome Induced by Simulated Viral Infection Using Synthetic RNA", CANNABIS AND CANNABINOID RESEARCH, vol. 5, no. 3, 1 September 2020 (2020-09-01), pages 197 - 201, XP055764826, ISSN: 2578-5125, DOI: 10.1089/can.2020.0043
- NGUYEN LONG CHI ET AL: "Cannabidiol Inhibits SARS-CoV-2 Replication and Promotes the Host Innate Immune Response", BIORXIV, 10 March 2021 (2021-03-10), XP055879584, Retrieved from the Internet <URL:https://www.ncbi.nlm.nih.gov/pmc/articles/PMC7987002/pdf/nihpp-2021.03.10.432967.pdf> [retrieved on 20220117], DOI: 10.1101/2021.03.10.432967
- WANG BO ET AL: "In search of preventive strategies: novel high-CBD Cannabis sativa extracts modulate ACE2 expression in COVID-19 gateway tissues", 22 November 2020 (2020-11-22), pages 22425 - 22444, XP093176812, Retrieved from the Internet <URL:https://www.aging-us.com/article/202225/pdf>
- DE CLERCQ ERIK ET AL: "Approved antiviral drugs over the past 50 years", CLINICAL MICROBIOLOGY REVIEW, WASHINGTON, DC, US, vol. 29, no. 3, 8 June 2016 (2016-06-08), pages 695 - 747, XP009528410, ISSN: 0893-8512, DOI: 10.1128/CMR.00102-15
- GIUSEPPE ESPOSITO; MARCELLA PESCE; LUISA SEGUELLA; WALTER SANSEVERINO; JIE LU; CHIARA CORPETTI; GIOVANNI SARNELLI: "The potential of cannabidiol in the COVID-19 pandemic", BRITISH JOURNAL OF PHARMACOLOGY, WILEY-BLACKWELL, UK, vol. 177, no. 21, 16 July 2020 (2020-07-16), UK , pages 4967 - 4970, XP071172311, ISSN: 0007-1188, DOI: 10.1111/bph.15157
- COSTINIUK CECILIA T.; JENABIAN MOHAMMAD-ALI: "Acute inflammation and pathogenesis of SARS-CoV-2 infection: Cannabidiol as a potential anti-inflammatory treatment?", CYTOKINE & GROWTH FACTOR REVIEWS, ELSEVIER LTD, GB, vol. 53, 20 May 2020 (2020-05-20), GB , pages 63 - 65, XP086180311, ISSN: 1359-6101, DOI: 10.1016/j.cytogfr.2020.05.008

(52) Cooperative Patent Classification (CPC): (Cont.)
A61K 38/21; A61K 45/06; A61P 11/00; A61P 31/14

C-Sets
A61K 31/13, A61K 2300/00;
A61K 31/215, A61K 2300/00;
A61K 31/46, A61K 2300/00;
A61K 31/4725, A61K 2300/00;
A61K 31/513, A61K 2300/00;
A61K 31/522, A61K 2300/00;
A61K 31/536, A61K 2300/00;
A61K 31/551, A61K 2300/00;
A61K 31/658, A61K 2300/00;
A61K 31/662, A61K 2300/00;
A61K 31/675, A61K 2300/00;
A61K 31/683, A61K 2300/00;
A61K 31/7012, A61K 2300/00;
A61K 31/7056, A61K 2300/00;
A61K 31/7072, A61K 2300/00;
A61K 38/21, A61K 2300/00

## Description

The present application is based on the application with the CN application number of 202010722697.9 and the filing date of July 24, 2020, and claims its priority.

## Technical Field

The present application relates to the field of chemical medicine, in particular to use of cannabidiol (structure of which is shown in Formula I), or a pharmaceutically acceptable salt, solvate or hydrate thereof, or a pharmaceutical composition thereof for inhibiting the replication and/or reproduction of SARS-CoV-2.

## Background Art

Coronavirus is an enveloped, unsegmented, single-stranded positive-stranded RNA virus that has a wide range of animal hosts. Coronavirus like SARS and MERS derived from animal infectious diseases can cause death in humans. On February 11, 2020, the International Committee on Taxonomy of Viruses (ICTV) announced a new coronavirus -- severe acute respiratory syndrome coronavirus 2 (SARS-CoV-2). At present, the novel coronavirus infection is mainly treated with supportive therapy in clinic, and no specific antiviral drug is available yet.

Cannabidiol (compound of Formula I) with chemical name of (-)-2-[(3R,4R)-p-mentha-1,8-dien-3β-yl]-5-pentylresorcinol is a cannabinoid compound with various biological activities. This drug has a significant therapeutic effect on epilepsy, and the anti-epileptic drug (Epidiolex) with cannabidiol as the active ingredient has been marketed in the United States.

Cannabidiol, first isolated from marijuana in 1940, is a non-psychoactive ingredient that is not hallucinogenic and does not cause physical dependence. In recent years, due to its wide range of biological effects, cannabidiol has received more and more attentions from the international community. Cannabidiol has a variety of pharmacological activities, and has certain therapeutic effects on neurological diseases including anxiety, schizophrenia, addiction, neurodegenerative diseases, etc. In addition, cannabidiol has pharmacological activities such as antiepileptic, anticonvulsant, antitumor, anticonvulsant, antiemetic, antidiabetic, and liver protection. Drugs, cosmetics, health care products and other products with cannabidiol as the main active ingredient have been marketed abroad and have broad application prospects.

At present, the antiviral activity of cannabidiol and related research on the treatment of viral diseases have not been fully carried out.

Costiniuk et al. discussed cannabidiol as a potential anti-inflammatory treatment for SARS-CoV-2 infection (Costiniuk, Cecilia T., and Mohammad-Ali Jenabian. "Acute inflammation and pathogenesis of SARS-CoV-2 infection: Cannabidiol as a potential anti-inflammatory treatment?." Cytokine & growth factor reviews 53 (2020): 63).

Esposito et al. suggested the hypothesis that cannabidiol has the potential to limit the severity and progression of Covid-19 (Esposito, Giuseppe, et al. "The potential of cannabidiol in the COVID-19 pandemic." British journal of pharmacology 177.21 (2020): 4967-4970).

## Contents of the Invention

The references to methods of treatment by therapy of this description are to be interpreted as references to compounds, pharmaceutical compositions and medicaments of the present invention for use in those methods. The invention is defined by the appended claims.

One object of the present application is to provide a medicament with inhibitory activity against a coronavirus, especially SARS-CoV-2, for the treatment of a disease or infection caused thereby, such as simple infection (e.g., fever, cough and sore throat, etc.), pneumonia, acute or severe acute respiratory infection, hypoxic respiratory failure, acute respiratory distress syndrome, sepsis or septic shock, etc. Studies have shown that cannabidiol can significantly reduce the viral nucleic acid load of cells infected with SARS-CoV-2 and has the function of inhibiting the replication of SARS-CoV-2. In some experiments, cannabidiol has $EC_{50}$ of 0.62 μM, $CC_{50}$ of 4.10 μM and SI of 6.61 against SARS-CoV-2 in Vero E6 cells, showing that it is very beneficial for the treatment of disease or infection caused by SARS-CoV-2.

Therefore, the present disclosure provides use of the compound of Formula I, or a pharmaceutically acceptable salt, solvate or hydrate thereof, or a pharmaceutical composition comprising any one or more of the above-mentioned components in the manufacture of a medicament for the prevention and/or treatment of a disease or infection caused by a coronavirus,

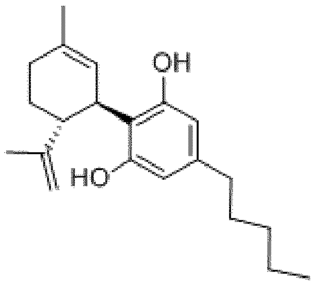

Formula I.

The present disclosure also provides the compound of Formula I, or a pharmaceutically acceptable salt, solvate or hydrate thereof, or a pharmaceutical composition comprising any one or more of the above components, which is used for the prevention and/or treatment of a disease or infection caused by a coronavirus,

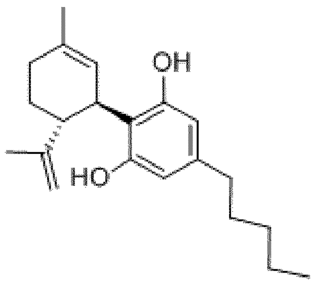

Formula I.

The present disclosure also provides a method for the prevention and/or treatment of a disease or infection caused by a coronavirus, comprising a step of administering to a subject in need thereof an effective amount of the compound of Formula I, or a pharmaceutically acceptable salt, solvate or hydrate thereof, or a pharmaceutical composition comprising any one or more of the above components,

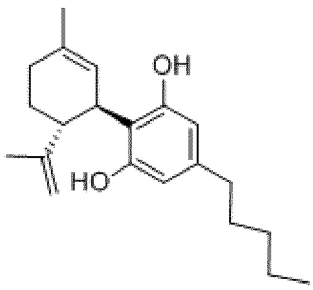

Formula I.

The present disclosure also provides use of the compound of Formula I, or a pharmaceutically acceptable salt, solvate or hydrate thereof, or a pharmaceutical composition comprising any one or more of the above-mentioned components in the manufacture of a medicament for the prevention and/or treatment of a respiratory disease,

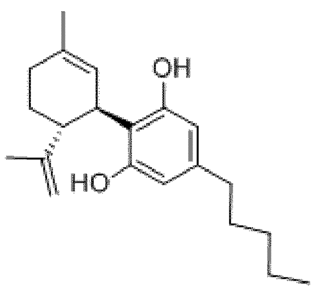

Formula I.

The present disclosure also provides the compound of Formula I, or a pharmaceutically acceptable salt, solvate or hydrate thereof, or a pharmaceutical composition comprising any one or more of the above-mentioned components, for use in a medicament for the prevention and/or treatment of a respiratory disease,

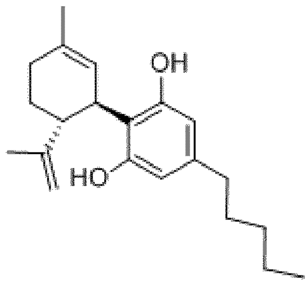

Formula I.

The present disclosure also provides a method for the prevention and/or treatment of a respiratory disease, comprising a step of administering to a subject in need thereof an effective amount of the compound of Formula I, or a pharmaceutically acceptable salt, solvate or hydrate thereof, or a pharmaceutical composition comprising any one or more of the above components,

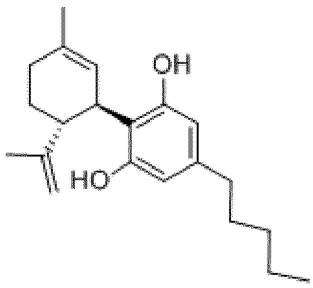

Formula I.

The present disclosure also provides use of the compound of Formula I, or a pharmaceutically acceptable salt, solvate or hydrate thereof, or a pharmaceutical composition comprising any one or more of the above-mentioned components in the manufacture of a coronavirus inhibitor,

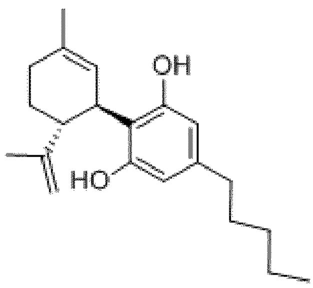

Formula I.

The present disclosure also provides the compound of Formula I, or a pharmaceutically acceptable salt, solvate or hydrate thereof, or a pharmaceutical composition comprising any one or more of the above-mentioned components, for use in inhibiting a coronavirus,

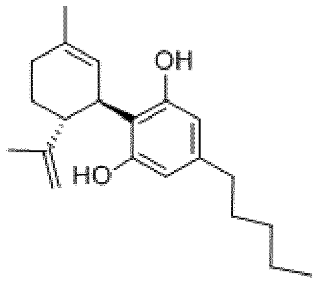

Formula I.

The present disclosure also provides a method for inhibiting a coronavirus, comprising a step of administering to a cell or subject in need thereof an effective amount of the compound of Formula I, or a pharmaceutically acceptable salt, solvate or hydrate thereof, or a pharmaceutical composition comprising any one or more of the above-mentioned components,

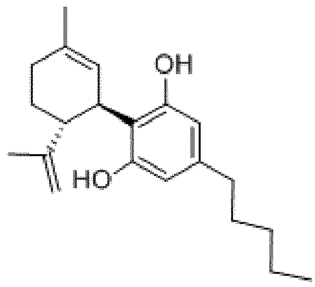

Formula I.

The present invention relates to the compound of Formula I, or a pharmaceutically acceptable salt, solvate or hydrate thereof, or a pharmaceutical composition comprising any one or more of the above-mentioned components, for use in inhibiting the replication and/or reproduction of SARS-CoV-2 in a cell in a subject or *in vivo,*

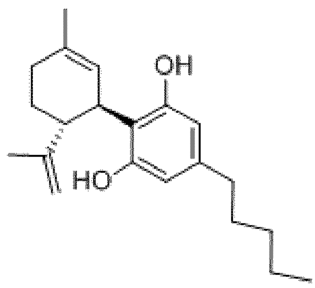

Formula I.

The present invention also relates to a method for inhibiting the replication and/or reproduction of SARS-CoV-2 *in vitro,* in a cell, comprising a step of contacting the cell with an effective amount of the compound of Formula I, or a pharmaceutically acceptable salt, solvate or hydrate thereof, or a pharmaceutical composition comprising any one or more of the above-mentioned components,

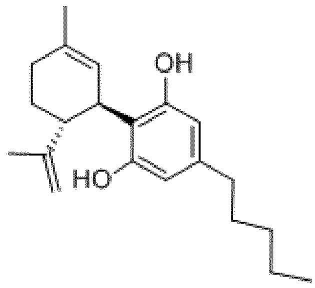

Formula I.

Clinical studies have found that the symptoms of SARS-CoV-2 infection are mainly pneumonia, which can be divided into simple infection, mild pneumonia, severe pneumonia, acute respiratory distress syndrome, sepsis, and septic shock, etc., according to the severity of the disease. Patients with simple infection may have nonspecific symptoms such as fever, cough, sore throat, nasal congestion, fatigue, headache, muscle pain or discomfort, and elderly and immunosuppressed individuals may experience atypical symptoms. The main symptoms of patients with mild pneumonia are cough, dyspnea and shortness of breath. Severe pneumonia can be seen in adolescents, adults, or children, and main symptoms are increased respiratory rate, severe respiratory failure or dyspnea, central cyanosis, lethargy, confusion or convulsions, gasp, etc. The lung images of acute respiratory distress syndrome are bilateral ground-glass opacities, which cannot be completely explained by effusion, lobar exudation, atelectasis, or pulmonary mass, with pulmonary edema as the main symptom. Patients with sepsis often have fatal organ dysfunction, and septic shock is the most critical patient with a high probability of death.

The disease or infection of any aspect herein is COVID-19.

In some embodiments, the cell of any aspect herein is a mammalian cell. In some embodiments, the mammal is selected from the group consisting of bovine, equine, ovine, porcine, canine, feline, rodent, and primate. In some embodiments, the mammal is human, cat, pig, or dog. In some embodiments, the mammal is a human.

The coronavirus of any aspect herein is SARS-CoV-2.

In some embodiments, the compound of Formula I, or a pharmaceutically acceptable salt, solvate or hydrate thereof in the pharmaceutical composition is the only active pharmaceutical component.

In some embodiments, the pharmaceutical composition further comprises an additional antiviral active ingredient.

In some embodiments, the compound of Formula I, or a pharmaceutically acceptable salt, solvate or hydrate thereof, or the pharmaceutical composition thereof, is administered in combination with the additional antiviral active ingredient. In some embodiments, the compound of Formula I, or a pharmaceutically acceptable salt, solvate or hydrate thereof is present in the same formulation unit with the additional antiviral active ingredient.

In some embodiments, the compound of Formula I, or a pharmaceutically acceptable salt, solvate or hydrate thereof, and the additional antiviral active ingredient are present in different formulation units.

In some embodiments, the compound of Formula I, or a pharmaceutically acceptable salt, solvate or hydrate thereof, is administered simultaneously, separately or sequentially with the additional antiviral active ingredient.

In some embodiments, the additional antiviral active ingredient is one or more selected from the group consisting of amantadine, rimantadine, enfuvirtide, maraviroc, acyclovir, ganciclovir, valacyclovir, famciclovir, foscarnet sodium, lamivudine, zidovudine, emtricitabine, tenofovir, adefovir dipivoxil, efavirenz, nevirapine, saquinavir, oseltamivir, zanamivir, ribavirin and interferon.

In some embodiments, the pharmaceutical composition may further comprise a pharmaceutically acceptable carrier or excipient. The carrier refers to a substance used to improve the selectivity, efficacy and/or safety of the drug during delivery. It is mainly used to control drug release, and can also be used to improve the pharmacokinetic properties of the drug, especially bioavailability. The excipient refers to substances other than the active ingredients in pharmaceutical formulation, which are mainly used for long-term stability, filling solid preparation (hence, also often used to specifically refer to "filler") or enhancing the efficacy of product (e.g., promoting absorption, reducing viscosity or increasing solubility, etc.). Depending on the route of administration or form of administration, those skilled in the art can select suitable carrier and excipient on the basis of known theory and experiences.

The pharmaceutical composition described herein can be prepared into various forms according to different routes of administration.

According to the present invention, the pharmaceutical composition can be administered in any of the following ways: oral administration, spray inhalation, rectal administration, nasal administration, buccal administration, vaginal administration, topical administration, parenteral administration such as subcutaneous, intravenous, intramuscular, intraperitoneal, intrathecal, intraventricular, intrasternal and intracranial injection or infusion, or via an explanted reservoir.

Of these, oral, intraperitoneal or intravenous administration is preferred.

For oral administration, the compound of Formula I, or a pharmaceutically acceptable salt, solvate or hydrate thereof, can be made into any orally acceptable preparation, including but not limited to tablet, capsule, aqueous solution or suspension. Among them, the commonly used carrier for tablet includes lactose and corn starch, and lubricant such as magnesium stearate may also be added. Commonly used diluent for capsule preparation includes lactose and dried cornstarch. Aqueous suspension is usually prepared by mixing the active ingredient with suitable emulsifying agent and suspending agent. If desired, some sweetening, flavoring or coloring agents may also be added to the above oral preparations.

For rectal administration, the compound of Formula I, or a pharmaceutically acceptable salt, solvate or hydrate thereof, can generally be made into the form of suppository, which is prepared by mixing the drug with a suitable non-irritating excipient. The excipient is solid at room temperature, but melts at rectal temperature to release the drug. Such excipient includes cocoa butter, beeswax and polyethylene glycol.

For topical administration, especially when treating affected surfaces or organs easily accessible by topical administration, such as eyes, skin or lower intestinal neurological diseases, the compound of Formula I, or a pharmaceutically acceptable salt, solvate or hydrate thereof, can be made into different topical preparations according to different affected surfaces or organs, and the specific instructions are as follows:

For topical administration to the eye, the compound of Formula I, or a pharmaceutically acceptable salt, solvate or hydrate thereof, the compound of Formula I, or pharmaceutically acceptable salt, solvate or hydrate thereof, can be formulated into the form of a micronized suspension or solution, the carrier used is isotonic sterile saline with a certain pH, which may or may not be added with a preservative such as benzyl alkoxide chloride. In addition, for ophthalmic use, the compound can be formulated into the form of an ointment such as petrolatum ointment.

For topical administration to the skin, the compound of Formula I, or a pharmaceutically acceptable salt, solvate or hydrate thereof, can be made into the form of a suitable ointment, lotion or cream, in which the active ingredient is suspended or dissolved in one or more carriers. The carriers that can be used in ointment here include, but are not limited to: mineral oil, liquid petrolatum, white petrolatum, propylene glycol, polyethylene oxide, polypropylene oxide, emulsified wax and water; the carriers that can be used in lotion or cream include, but are not limited to: mineral oil, sorbitan monostearate, Tween 60, cetyl ester wax, cetenylaryl alcohol, 2-octyldodecanol, benzyl alcohol and water.

For topical administration to the lower intestinal tract, the compound of Formula I, or a pharmaceutically acceptable salt, solvate or hydrate thereof, can be made into the above-mentioned rectal suppository preparation or suitable form of enema preparation. In addition, topical transdermal patches can also be used.

The compound of Formula I, or a pharmaceutically acceptable salt, solvate or hydrate thereof, can also be administered in the form of sterile injectable preparation, including sterile injectable aqueous or oil suspension, or sterile injectable solution. Among them, the carriers and solvents that can be used are water, Ringer's solution and isotonic sodium chloride solution. In addition, sterile nonvolatile oil such as monoglyceride or diglyceride can also be used as a solvent or suspending medium.

The medicament of any one of the above-mentioned various dosage forms can be prepared according to the conventional methods in the pharmaceutical field.

In some embodiments, the pharmaceutical composition is a solid preparation or a liquid preparation. In some embodiments, the pharmaceutical composition is a tablet, injection or spray. In some embodiments, the pharmaceutical composition is an injection.

In the present application, unless otherwise indicated, the scientific and technical terms used herein have the meanings commonly understood by those skilled in the art. Also, the procedures of cell culture, molecular genetics, nucleic acid chemistry, immunology laboratory operation used herein are all conventional and widely used in the corresponding fields. Meanwhile, for the purpose of better understanding of the present invention, the definitions and explanations of related terms are provided below.

As used herein, the term "pharmaceutically acceptable salt" includes an inorganic acid salt or an organic acid salt, and an inorganic base salt or an organic base salt, such as sodium salts, potassium salts, calcium salts, lithium salts, meglumine salt, hydrochloride salts, hydrobromide salts, hydroiodide salts, nitrates, sulfates, phosphates, hydrogen phosphates, acetates, propionates, butyrates, oxalates, trimethyl acetates, adipates, alginates, lactates, citrates, tartrates, succinates, maleates, fumarates, picrates, aspartates, gluconates, benzoates, methanesulfonates, ethanesulfonates, benzenesulfonates, p-toluenesulfonates or pamoates, etc..

A compound of formula I in the disclosure may exist in the form of a solvate (preferably a hydrate), and it comprises a polar solvent, especially water, methanol or ethanol, as a structural element of the lattice. The amount of the polar solvent is stoichiometrical or non-stoichiometrical. It shall be understood that, even though the solvates of the compound of formula I used in the treatment of the disease or infection defined in the present application may have different properties (including pharmacokinetic properties), once absorbed in the subject, the compound of formula I will be obtained, so that the use of the compound of formula I covers the use of any solvate of the compound of formula I.

In the present application, the official classification name of the term "2019 novel coronavirus (2019-nCoV)" is

severe acute respiratory syndrome coronavirus 2 (SARS-CoV-2).

In the present application, the term "disease caused by the 2019 novel coronavirus (2019-nCoV)" is officially named as COVID-19.

In this application, the term "therapeutically effective amount" or "prophylaxically effective amount" refers to, within the scope of reasonable medical judgment, an amount sufficient to treat or prevent the patient's disease but low enough to avoid serious side effects (at a reasonable benefit/risk ratio). The therapeutically effective amount of the compound may vary based on factors including the specific compound selected (for example, considering the potency, effectiveness and half-life of the compound), the selected route of administration, the disease to be treated, the severity of the disease to be treated, age, size, weight, and physical disease of the patient being treated, the medical history of the patient being treated, the duration of treatment, the nature of concurrent therapy, the desired therapeutic effect, and etc., but it can still be routinely determined by those skilled in the art.

In addition, it should be pointed out that the specific dosage and route of administration of the compound of formula I, or a a pharmaceutically acceptable salt, a solvate or a hydrate thereof are determined by many factors, including the age of the patient, body weight, gender, health status, nutritional status, potency of the drug, time taken, metabolic rate, severity of the disease, and the subjective judgment of the physician. Preferable dose is between 0.001-1000 mg/kg body weight/day.

## Brief Description of the Drawings

The description of the drawings herein is provided for further explanation of the present invention, and constitutes part of the present application. The exemplary embodiments and description are meant to explain the present invention, which is defined by the claims.

In the drawings:

Fig. 1 shows the effect of cannabidiol on viral nucleic acid load on SARS-CoV-2-infected vero E6 cells; cannabidiol was able to inhibit viral RNA load on cells 48h after the cells were infected with SARS-CoV-2, and the inhibitory activity was dose-dependent. Wherein, the left ordinate represents the percentage inhibition rate calculated based on the number of copies of viral RNA in the sample (corresponding to the dots and fitting line thereof in the figure), the right ordinate represents the percentage toxicity calculated based on cell viability (corresponding to the squares and fitting line thereof in the figure), and the abscissa represents the drug concentration.

## Specific Models for Carrying Out the Invention

The technic solutions of the embodiments of the present invention is further illustrated clearly and completely in the following examples and the drawings. Obviously, they are merely part, and not all of the examples. The invention is defined by the appended claims.

Example 1: Experiment of cannabidiol in reduction of viral nucleic acid load of cells infected by SARS-CoV-2

(1) Drug treatment of virus-infected cells

Vero E6 cells (purchased from ATCC, Catalog No. 1586) were placed into a 24-well plate and incubated for 24 hours, then virus infection was carried out, specifically, SARS-CoV-2 (2019-nCoV) virus (nCoV-2019BetaCoV/Wuhan/-WIV04/2019 strain, provided by Wuhan Institute of Virology, Chinese Academy of Sciences) was diluted with 2% cell maintenance solution (formulation: FBS (purchased from Gibco, Catalog No.: 16000044) was added to MEM (purchased from Gibco, Article No: 10370021) by a volume ratio of 2%, thereby obtaining the 2% cell maintenance solution) to corresponding concentration, and then added to the 24-well plate so that each well contained a viral load of 100$TCID_{50}$. Next, cannabidiol (purchased from Selleck Chemicals, Article No.: S7975) was diluted with 2% cell maintenance solution to the corresponding concentrations and added to corresponding wells, so that the final drug concentrations were 100 μM, 33 μM, 11 μM, 3.7 μM, 1.23 μM, 0.41 μM, 0.14 μM, respectively, then the plate was put in 37°C, 5% $CO_2$ incubator and continuously cultured for 48h, and the cell vehicle control group was added with only 2% cell maintenance solution without any test drug.

(2) RNA extraction

The RNA extraction kit was purchased from Qiagen, Article No.: 74106. The consumptive materials (spin column, RNase-free 2ml collection tube, etc.) and reagents (RLT, RW1, RPE, RNase-free water, etc.) involved in the following RNA extraction steps were all parts of the kit. The following extraction steps were all recommended by the kit instructions.

1) 100 μL of the supernatant was taken from the test plate, added to a nuclease-free EP tube, then added with 350 μL of Buffer RLT, mixed by a transfer liquid gun to make it fully lysed, and centrifuged to take the supernatant;

2) the supernatant obtained in step 1) was added with an equal volume of 70% ethanol and mixed well;

3) the mixed solution obtained in step 2) above was transferred to a RNase-free spin column, centrifuged at 12000 rpm for 15s, and the waste liquid was discarded;

4) 700μL of Buffer RW1 was added to the spin column, then centrifugation was carried out at 12000 rpm for 15s to clean the spin column, and the waste liquid was discarded;

5) 500μL of Buffer RPE was added to the spin column, then centrifugation was carried out at 12000 rpm for 15s to clean the spin column, and the waste liquid was discarded;

6) 500μL of Buffer RPE was added to the spin column, then centrifugation was carried out at 12000 rpm for 2min to clean the spin column, and the waste liquid was discarded;

7) the spin column was placed in a new RNase-free 2 ml collection tube, and centrifugation was carried out at 12000 rpm for 1 min to dry the spin column, and then the entire spin column was transferred to the 1.5 ml collection tube of step 8);

8) the spin column dried in step 7) was placed in a new 1.5ml collection tube, added with $30_{11}1$ of RNase-free water, and centrifuged at 12000 rpm for 2min, the obtained eluent contained the corresponding RNA, and was added with RNase inhibitor (purchased from NEB, Article No.: M0314L), and detected with Nano Drop (purchased from Thermo scientific, Nano Drop One) to determine each RNA concentration.

(3) RNA reverse transcription

In the experiment, the reverse transcription kit (PrimeScript™ RT reagent Kit with gDNA Eraser, Catalog No. RR047Q) produced by TaKaRa Company was used for RNA reverse transcription. The steps were as follows.

1) gDNA removal: RNA samples from each experimental group were collected, and 1 μg thereof was taken and subjected to reverse transcription. First, 2 μl of 5× gDNA Eraser Buffer was added to the RNA sample of each experimental group, the reaction system was supplemented with RNase Free water to 10 μl, mixed well, and subjected to 42 °C water bath for 2 min to remove the gDNA that might exist in the sample;

2) Reverse transcription: the sample obtained in 1) was added with appropriate amounts of enzyme, primer Mix and reaction buffer, supplemented with RNase Free water to an volume of 20 μl, reacted under 37°C water bath for 15 min, then put in 85°C water bath for 5 sec, thereby obtaining cDNA via transcription.

(4) Real-time PCR

Fluorescence quantitative PCR was used to detect the copy number per ml of the original virus solution.

The reaction system was mixed using TB Green Premix (Takara, Cat#RR820A), and the amplification reaction and reading were carried out with StepOne Plus Real-time PCR instrument (brand: ABI). The copy number contained in per ml of the original virus solution was calculated. The steps were as follows:

1) Establishment of standards: the plasmid pMT-RBD (the plasmid was provided by Wuhan Institute of Virology, Chinese Academy of Sciences) was diluted to $5\times10^8$ copies/μL, $5\times10^7$ copies/μL, $5\times10^6$ copies/μL, $5\times10^5$ copies/μL, $5\times10^4$ copies/μL, $5\times10^3$ copies/μL, $5\times10^2$ copies/μL. 2 μL standard or cDNA template was taken for qPCR reaction.

2) The sequences of primers used in the experiment were as follows (all indicated in 5'-3' direction):

RBD-qF: CAATGGTTTAACAGGCACAGG

RBD-qR: CTCAAGTGTCTGTGGATCACG

3) The reaction procedure was as follows:

Pre-denaturation: 95 °C for 5 minutes;
Cycle parameters: 95 °C for 15 seconds, 54 °C for 15 seconds, 72 °C for 30 seconds, for a total of 40 cycles.

(5) Cytotoxicity test of drug

The detection of the drug cytotoxicity was performed using CCK-8 kit (Beoytime). Specific steps were as follows:

1) $1\times10^4$ Vero E6 (ATCC) cells were placed in a 96-well plate and incubated at 37°C for 8 hours.

2) The drug was diluted with DMSO to an appropriate concentration of mother liquor, and then diluted with MEM medium (purchased from Gibco, Catalog No. 10370021) containing 2% FBS (purchased from Gibco, Catalog No. 16000044) to the same concentration as that for the drug treatment. The original medium in the 96-well plate was discarded, 100 μL of drug-containing MEM medium was added to the cells, and three replicate wells were prepared for each concentration. Negative control (vehicle group, DMSO and medium were added to the cell wells, without adding drug) and blank control (DMSO and medium were added to the wells, without cells) were set up. After the drug was added, the cells were cultured at 37°C for 48 hours.

3) 20 μL of CCK-8 solution (Beoytime) was added to the well to be tested, mixed gently, without generating bubbles, and continuously incubated at 37 °C for 2 hours. $OD_{450}$ was read on a microplate reader (purchased from Molecular Devices, Model: SpectraMax M5), and cell viability was calculated:

$$\text{Cell activity (\%)} = (A_{(\text{drug treatment group})} - A_{(\text{blank control})}) / (A_{(\text{vehicle control})} - A_{(\text{blank control})}) \times 100\%$$

wherein A was the reading of the microplate reader.

(6) Experimental results

The results of the virus proliferation inhibition experiment showed that the test compound at concentrations of 10 μM, 3.3 μM, 1.1 μM and 0.37 μM could effectively inhibit the replication of the SARS-CoV-2 virus genome in the infected supernatant (Table 1 and Fig. 1).

Table 1. Antiviral experiments of test compound (cannabidiol)

| Concentration (μM) | Viral genome copy number (MOI = 0.05) |
|---|---|
| 10 | 532013±16451 |
| 3.33 | 668555500±83251338 |
| 1.11 | 877127896±122398434 |
| 0.37 | 1224296104±183149682 |
| 0.12 | 1860569042±365350110 |
| 0.04 | 1853011000±279114748 |
| Vehicle | 2215912896±388319129 |

The cytotoxicity results showed that the treatment by the test compound (cannabidiol) did not change the cell viability at concentrations of 1.56 μM and 0.78 μM, that was, the test compound had no toxic effect on the cells at all concentrations (Table 2 and Fig. 1).

Table 2. Cytotoxicity assay of test compound of (cannabidiol)

| Concentration (μM) | Cell viability (%) |
|---|---|
| 100 | 8.87±3.59 |
| 50 | 22.85±18.47 |
| 25 | 38.39±5.70 |

(continued)

| Concentration (μM) | Cell viability (%) |
|---|---|
| 12.5 | 50.01±8.40 |
| 6.25 | 60.07±6.01 |
| 3.13 | 58.45±5.30 |
| 1.56 | 74.22±13.42 |
| 0.78 | 73.45±7.48 |
| Vehicle | 100±3.21 |

## Claims

1. The compound of Formula I, or a pharmaceutically acceptable salt, solvate or hydrate thereof, or a pharmaceutical composition comprising any one or more of the above-mentioned components for use in inhibiting the replication and/or reproduction of SARS-CoV-2 in a cell in a subject,

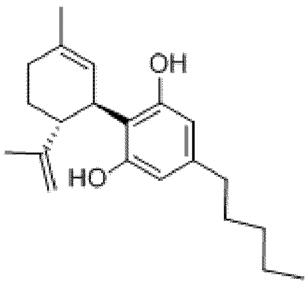

I.

2. The compound of Formula I, or a pharmaceutically acceptable salt, solvate or hydrate thereof, or a pharmaceutical composition comprising any one or more of the above-mentioned components for use in inhibiting the replication and/or reproduction of SARS-CoV-2 in a cell *in vivo,*

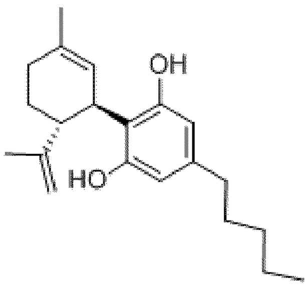

I.

3. A method of inhibiting the replication and/or reproduction of SARS-CoV-2 *in vitro,* comprising contacting a cell with an effective amount of the compound of Formula I, or a pharmaceutically acceptable salt, solvate or hydrate thereof, or a pharmaceutical composition comprising any one or more of the above-mentioned components,

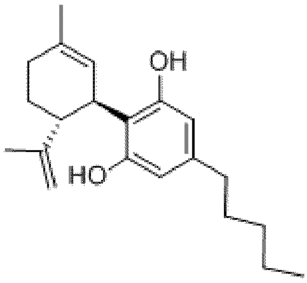

I.

**4.** The method according to claim 3, wherein the cell is a mammalian cell.

**5.** The method according to claim 4, wherein, the mammalian cell is a human cell.

**6.** The compound of Formula I, or a pharmaceutically acceptable salt, solvate or hydrate thereof, or a pharmaceutical composition comprising any one or more of the above-mentioned components for use according to claims 1 or 2, or the method according to any one of claims 3-5, , wherein the pharmaceutical composition further comprises a pharmaceutically acceptable carrier or excipient.

**7.** The compound of Formula I, or a pharmaceutically acceptable salt, solvate or hydrate thereof, or a pharmaceutical composition comprising any one or more of the above-mentioned components for use according to claims 1 or 2, or the method according to any one of claims 3-5, , wherein, the pharmaceutical composition is a tablet, an injection or a spray.

**8.** The compound of Formula I, or a pharmaceutically acceptable salt, solvate or hydrate thereof, or a pharmaceutical composition comprising any one or more of the above-mentioned components for use according to any one of claims 1, 2, 6 and 7, or the method according to any one of claims 3-7, wherein the compound of Formula I, or a pharmaceutically acceptable salt, solvate or hydrate thereof is used as the only pharmaceutically active ingredient in the pharmaceutical composition;
or, the pharmaceutical composition further comprises an additional antiviral active ingredient.

**9.** The compound of Formula I, or a pharmaceutically acceptable salt, solvate or hydrate thereof, or a pharmaceutical composition comprising any one or more of the above-mentioned components for use according to claim 8, or the method according to claim 8, wherein, the compound of Formula or a pharmaceutically acceptable salt, solvate or hydrate thereof, or the pharmaceutical composition is administered in combination with the additional antiviral active ingredient by simultaneous, separate or sequential administration.

**10.** The compound of Formula I, or a pharmaceutically acceptable salt, solvate or hydrate thereof, or a pharmaceutical composition comprising any one or more of the above-mentioned components for use according to claim 8, or the method according to claim 8, wherein, the additional antiviral active ingredient is one or more selected from the group consisting of amantadine, rimantadine, enfuvirtide, maraviroc, acyclovir, ganciclovir, valacyclovir, famciclovir, foscarnet sodium, lamivudine, zidovudine, emtricitabine, tenofovir, adefovir dipivoxil, efavirenz, nevirapine, saquinavir, oseltamivir, zanamivir, ribavirin and interferon.

**Patentansprüche**

**1.** Verbindung der Formel I oder pharmazeutisch verträgliches Salz, Solvat oder Hydrat davon oder pharmazeutische Zusammensetzung, welche einen oder mehrere der vorstehend genannten Bestandteile umfasst, zur Verwendung bei der Hemmung der Replikation und/oder der Reproduktion von SARS-COV-2 in einer Zelle in einem Subjekt,

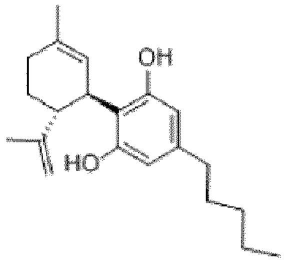

I.

2. Verbindung der Formel I oder pharmazeutisch verträgliches Salz, Solvat oder Hydrat davon oder pharmazeutische Zusammensetzung, welche einen oder mehrere der vorstehend genannten Bestandteile umfasst, zur Verwendung bei der Hemmung der Replikation und/oder der Reproduktion von SARS-COV-2 in einer Zelle *in vivo,*

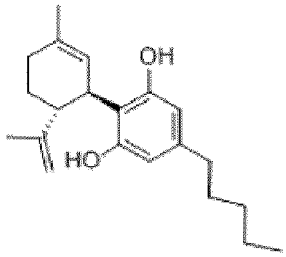

I.

3. Verfahren zur Hemmung der Replikation und/oder der Reproduktion von SARS-COV-2 *in vitro,* umfassend ein Kontaktieren einer Zelle mit einer wirksamen Menge der Verbindung der Formel I oder eines pharmazeutisch verträglichen Salzes, Solvats oder Hydrats davon oder einer pharmazeutischen Zusammensetzung, welche einen oder mehrere der vorstehend genannten Bestandteile umfasst,

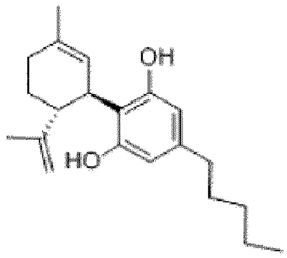

I.

4. Verfahren nach Anspruch 3, wobei die Zelle eine Säugerzelle ist.

5. Verfahren nach Anspruch 4, wobei die Säugerzelle eine menschliche Zelle ist.

6. Verbindung der Formel I oder pharmazeutisch verträgliches Salz, Solvat oder Hydrat davon oder pharmazeutische Zusammensetzung, welche einen oder mehrere der vorstehend genannten Bestandteile umfasst, zur Verwendung gemäß den Ansprüchen 1 oder 2 oder dem Verfahren nach einem der Ansprüche 3-5, wobei die pharmazeutische Zusammensetzung ferner einen pharmazeutisch verträglichen Trägerstoff oder Hilfsstoff umfasst.

7. Verbindung der Formel I oder pharmazeutisch verträgliches Salz, Solvat oder Hydrat davon oder pharmazeutische Zusammensetzung, welche einen oder mehrere der vorstehend genannten Bestandteile umfasst, zur Verwendung gemäß den Ansprüchen 1 oder 2 oder dem Verfahren nach einem der Ansprüche 3-5, wobei die pharmazeutische Zusammensetzung eine Tablette, eine Injektion oder ein Spray ist.

8. Verbindung der Formel I oder pharmazeutisch verträgliches Salz, Solvat oder Hydrat davon oder pharmazeutische

Zusammensetzung, welche einen oder mehrere der vorstehend genannten Bestandteile umfasst, zur Verwendung gemäß einem der Ansprüche 1, 2, 6 und 7 oder dem Verfahren nach einem der Ansprüche 3-7, wobei die Verbindung der Formel I oder ein pharmazeutisch verträgliches Salz, Solvat oder Hydrat davon als der einzige pharmazeutisch wirksame Bestandteil in der pharmazeutischen Zusammensetzung verwendet wird;
oder die pharmazeutische Zusammensetzung ferner einen zusätzlichen antiviralen Wirkstoff umfasst.

**9.** Verbindung der Formel I oder pharmazeutisch verträgliches Salz, Solvat oder Hydrat davon oder pharmazeutische Zusammensetzung, welche einen oder mehrere der vorstehend genannten Bestandteile umfasst, zur Verwendung gemäß Anspruch 8 oder dem Verfahren nach Anspruch 8, wobei die Verbindung der Formel I oder ein pharmazeutisch verträgliches Salz, Solvat oder Hydrat davon oder die pharmazeutische Zusammensetzung durch eine gleichzeitige, separate oder sequenzielle Verabreichung in Kombination mit dem antiviralen Wirkstoff verabreicht wird.

**10.** Verbindung der Formel I oder pharmazeutisch verträgliches Salz, Solvat oder Hydrat davon oder pharmazeutische Zusammensetzung, welche einen oder mehrere der vorstehend genannten Bestandteile umfasst, zur Verwendung gemäß Anspruch 8 oder dem Verfahren nach Anspruch 8, wobei der zusätzliche antivirale Wirkstoff eines oder mehrere ist, ausgewählt aus der Gruppe, bestehend aus Amantadin, Rimantadin, Enfuvirtide, Maraviroc, Acyclovir, Ganciclovir, Valacyclovir, Famciclovir, Foscarnet-Natrium, Lamivudin, Zidovudin, Emtricitabin, Tenofovir, Adefovir Dipivoxil, Efavirenz, Nevirapin, Saquinavir, Oseltamivir, Zanamivir, Ribavirin und Interferon.

## Revendications

**1.** Composé de formule I, ou sel, solvate ou hydrate pharmaceutiquement acceptable de celui-ci, ou composition pharmaceutique comprenant un ou plusieurs des composants susmentionnés, pour une utilisation destinée à inhiber la réplication et/ou la reproduction du SARS-CoV-2 dans une cellule chez un sujet,

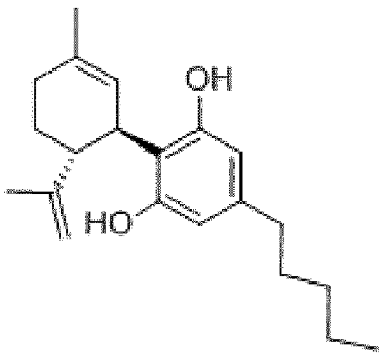

I.

**2.** Composé de formule I, ou sel, solvate ou hydrate pharmaceutiquement acceptable de celui-ci, ou composition pharmaceutique comprenant un ou plusieurs des composants susmentionnés, pour une utilisation destinée à inhiber la réplication et/ou la reproduction du SARS-CoV-2 dans une cellule *in vivo,*

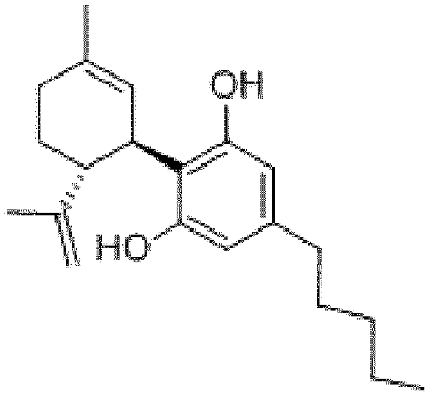

I.

3. Procédé d'inhibition de la réplication et/ou de la reproduction du SARS-CoV-*2 in vitro,* comprenant la mise en contact d'une cellule avec une quantité efficace du composé de formule I, ou d'un sel, solvate ou hydrate pharmaceutiquement acceptable de celui-ci, ou d'une composition pharmaceutique comprenant un ou plusieurs des composants susmentionnés,

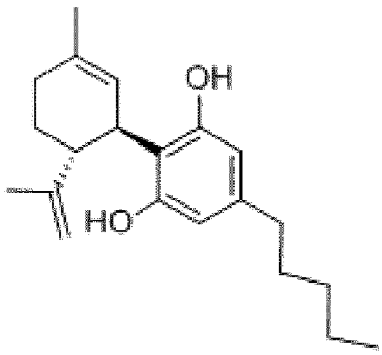

I.

4. Procédé selon la revendication 3, dans lequel la cellule est une cellule de mammifère.

5. Procédé selon la revendication 4, dans lequel la cellule de mammifère est une cellule humaine.

6. Composé de formule I, ou sel, solvate ou hydrate pharmaceutiquement acceptable de celui-ci, ou composition pharmaceutique comprenant un ou plusieurs des composants susmentionnés, pour une utilisation selon les revendications 1 ou 2, ou procédé selon l'une des revendications 3 à 5, dans lequel la composition pharmaceutique comprend en outre un véhicule ou excipient pharmaceutiquement acceptable.

7. Composé de formule I, ou sel, solvate ou hydrate pharmaceutiquement acceptable de celui-ci, ou composition pharmaceutique comprenant un ou plusieurs des composants susmentionnés, pour une utilisation selon les revendications 1 ou 2, ou procédé selon l'une des revendications 3 à 5, dans lequel la composition pharmaceutique est un comprimé, une solution injectable ou un spray.

8. Composé de formule I, ou sel, solvate ou hydrate pharmaceutiquement acceptable de celui-ci, ou composition pharmaceutique comprenant un ou plusieurs des composants susmentionnés, pour une utilisation selon l'une des revendications 1, 2, 6 et 7, ou procédé selon l'une des revendications 3 à 7, dans lequel le composé de formule I, ou un sel, solvate ou hydrate pharmaceutiquement acceptable de celui-ci, est utilisé comme seul principe actif pharmaceutiquement dans la composition pharmaceutique ; ou la composition pharmaceutique comprend en outre un principe actif antiviral supplémentaire.

9. Composé de formule I, ou sel, solvate ou hydrate pharmaceutiquement acceptable de celui-ci, ou composition

pharmaceutique comprenant un ou plusieurs des composants susmentionnés, pour une utilisation selon la revendication 8, ou procédé selon la revendication 8, dans lequel le composé de formule I, ou un sel, solvate ou hydrate pharmaceutiquement acceptable de celui-ci, ou la composition pharmaceutique est administré en association avec le principe actif antiviral supplémentaire par administration simultanée, séparée ou séquentielle.

**10.** Composé de formule I, ou sel, solvate ou hydrate pharmaceutiquement acceptable de celui-ci, ou composition pharmaceutique comprenant un ou plusieurs des composants mentionnés ci-dessus, pour une utilisation selon la revendication 8, ou procédé selon la revendication 8, dans lequel le principe actif antiviral supplémentaire est un ou plusieurs ingrédients choisis dans le groupe constitué par l'amantadine, la rimantadine, l'enfuvirtide, le maraviroc, l'acyclovir, le ganciclovir, le valacyclovir, le famciclovir, le foscarnet sodique, la lamivudine, la zidovudine, l'emtricitabine, le ténofovir, l'adéfovir dipivoxil, l'éfavirenz, la névirapine, le saquinavir, l'oseltamivir, le zanamivir, la ribavirine et l'interféron.

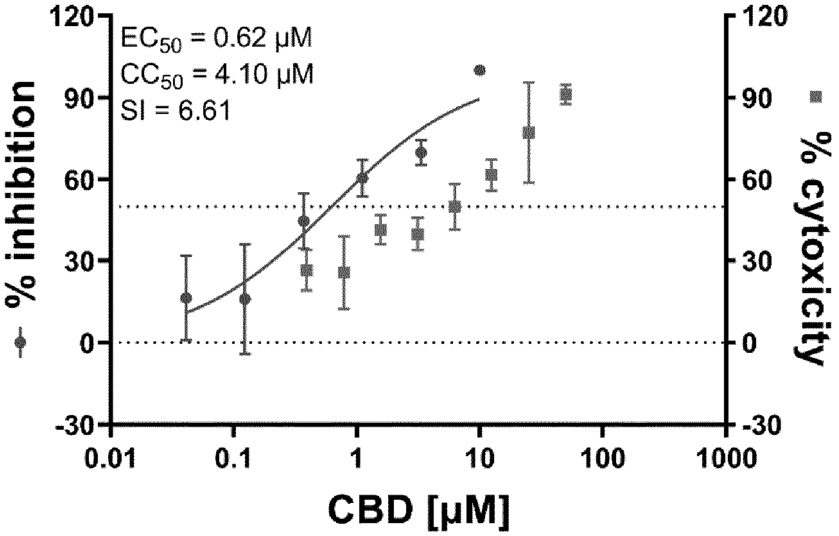
120
90
60
30
0
-30
% inhibition
EC$_{50}$ = 0.62 µM
CC$_{50}$ = 4.10 µM
SI = 6.61
120
90
60
30
0
-30
% cytoxicity
0.01
0.1
1
10
100
1000
CBD [µM]

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010722697


### Non-patent literature cited in the description


- **COSTINIUK, CECILIA T.** ; **MOHAMMAD-ALI JENABIAN**. Acute inflammation and pathogenesis of SARS-CoV-2 infection: Cannabidiol as a potential anti-inflammatory treatment?.. *Cytokine & growth factor reviews*, 2020, vol. 53, 63
- **ESPOSITO, GIUSEPPE et al.** The potential of cannabidiol in the COVID-19 pandemic.. *British journal of pharmacology*, 2020, vol. 177 (21), 4967-4970